# FASCICULE DE BREVET EUROPEEN

(11) **EP 1 165 581 B1**
(45) Date de publication et mention de la délivrance du brevet: **15.01.2003**
(21) Numéro de dépôt: 00915233.1
(22) Date de dépôt: 29.03.2000
(51) Int. Cl.: C07H 15/207, C07H 17/04

(54) **PROCEDE DE PREPARATION DU 4'-DEMETHYL -4'- PHOSPHATE-2",3" - BIS PENTAFLUOROPHENOXYACETYL -4",6" -ETHYLIDENE - BETA-D-GLUCOSIDE D'EPIPODOPHYLLOTOXINE, ET DE SES SELS**
VERFAHREN ZUR HERSTELLUNG VON EPIPODOPHYLLOTOXINE-4'-DEMETHYL-4'-PHOSPHAT-2",3"-BIS PENTAFLUORPHENOXYACETYL-4",6"-ETHYLIDEN- BETA-D-GLUCOSID UND SEINE SALZEN
METHOD FOR THE PRODUCTION OF 4'-DEMETHYL - 4'-PHOSPHATE-2",3" - BIS PENTAFLUOROPHENOXYACETYL - 4",6"-ETHYLIDENE - BETA-D- GLUCOSIDE EPIPODOPHYLLOTOXIN AND THE SALTS THEREOF

(30) Priorité: 29.03.1999 FR 9903877
(43) Date de publication de la demande: 02.01.2002
(73) Titulaire: PIERRE FABRE MEDICAMENT, 92100 Boulogne (FR)
(72) Inventeur: GUMINSKI, Yves, F-81090 Lagarrigue (FR); IMBERT, Thierry, F-81290 Viviers les Montagnes (FR)
(74) Mandataire: Ahner, Francis
(86) Numéro de dépôt international: FR0000776
(87) Numéro de publication internationale: WO00058324

(56) Documents cités:
- EP-A- 0 445 021
- WO-A-96/12727

## Description

La présente invention a pour objet un nouveau procédé de préparation du 4'-déméthyl-4'-phosphate-2",3"-bis pentafluorophénoxyacétyl-4",6"-éthylidène-β-D-glucoside d'épipodophyllotoxine et de ses sels, en particulier le sel de N-métnyl-D-glucamine.

Il consiste à préparer le 1-O-triéthyl silyl-2,3-dipentafluorophénoxy acétyl-4,6-éthylidène-β-D-glucoside et de le condenser avec le 4'-aryloxyacétyl-4'-déméthyl épipodophyllotoxine. Ce procédé a l'avantage d'utiliser un intermédiaire glucoside stable et cristallin et de former un composé de couplage dans un très bon état de pureté pour conduire, après phosphorylation en position 4', au composé 4'-déméthyl-4'-phosphate-2",3"-bis pentafluorophénoxyacétyl-4",6"-éthylidène-β-D-glucoside d'épipodophyllotoxine dont le sel de méthylglucamine, soluble dans l'eau, est utilisé comme agent anticancéreux.

Le 4'-déméthyl-4'-phosphate-2",3"-bispentafluorophénoxyacétyl-4",6"-éthylidène-β-D-glucoside d'épipodophyllotoxine de formule 1 sous forme de sel de méthylglucamine est un composé anticancéreux dont la structure, la préparation et son activité pharmacologique ont été montrées et publiées dans le brevet WO 96/12727.

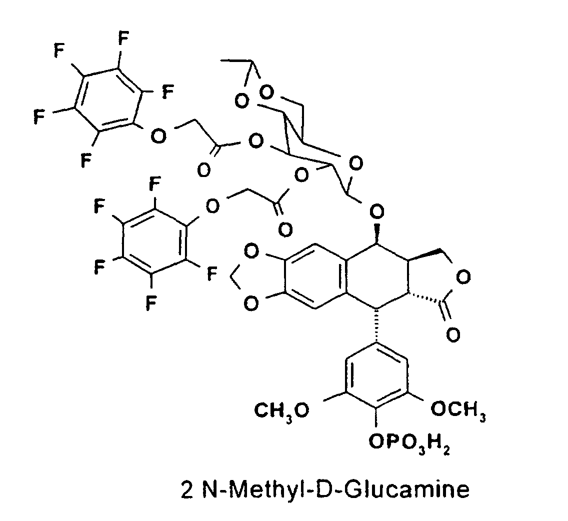

Ce composé est constitué par une partie lignane de type épipodophyllotoxine et d'une partie osidique en particulier le 4,6-éthylidène-2,3-bis pentafluorophénoxyacétyl-β-D-glupyranose. La position 4' porte un groupement phosphate qui lui confère à la fois une solubilité aqueuse par formation d'un sel avec, par exemple la N-méthylglucamine, mais également une activité pharmacologique très importante. La synthèse de ce composé décrite dans le brevet WO 96/12727 se faisait par un procédé en 12 étapes, utilisant l'étoposide comme intermédiaire de synthèse. L'étoposide lui-même était préparé selon un procédé en 6 stades qui a été breveté dans EP 445021. Ce procédé faisait intervenir le 1-O-carbobenzyloxy-2,3-bisphénoxyacétyl-4,6-éthylidène-β-D-glucopyranoside, lequel après hydrogénolyse et par couplage avec le 4'-phénoxyacétyl-4'-déméthylépipodophyllotoxine fournissait, après déprotection des groupements phénoxyacétyle, l'étoposide. Cet étoposide intermédiaire subissait encore un traitement en 6 stades, consistant en une protection en 4' par un groupement carbobenzyloxy (Z), puis estérification avec le chlorure de pentafluorophénoxyacétyle, suivi de l'hydrogénolyse du groupement Z en 4'. La fonction phosphate était introduite par l'action du POCl₃ suivi d'une hydrolyse. On obtenait alors après purification le dérivé phosphate permettant sa salification avec la N-Méthylglucamine pour la solubilité aqueuse et pour son activité.

Cette méthode est longue et nécessite de nombreux stades de synthèse sources d'impuretés, ainsi qu'une étape d'hydrogénolyse à un stade précoce de la synthèse, ce qui demande de traiter des quantités importantes de produit et de catalyseur précieux comme le Palladium. De plus, cette étape d'hydrogénolyse fournissait un composé, bien que de stabilité suffisante, qui devait être engagé sans trop attendre dans l'étape du couplage, pour éviter une anomérisation, qui conduirait inévitablement à la présence de l'isomère a glucoside.

La silylation des alcools anomères de sucres est bien connue pour fournir des alcools anomères silylés en position β exclusivement voir Chem. Ber. (1964), 97, 2196, où les auteurs préparent le triméthylsilyl tétraacétylglucose en tant qu'anomère β pur. Ceci a été repris également par Tietze ((Synthesis (1982), 946)). De même, Tet. Let (1991), 32, 2079, indique la préparation de sucres silylés en position anomérique sous forme d'anomère β de façon prédominante. Ces sucres silylés ont été utilisés pour des réactions de glycosidation en présence de triméthylsilyl triflate ou d'éthérate de BF₃, par exemple dans Tet. Let (1982), 23, 4661, ainsi que dans Synth. Comm. (1989), 19, 3453.

La présente invention concerne la préparation du 4'-déméthyl-4'-phosphate-. 2",3"-bispentafluorophénoxyacétyl-4",6"-éthylidène-β-D-glucoside d'épipodophyflotoxine et de ses sels, en particulier le sel de N-méthyl-D-glucamine. Cette synthèse consiste en la préparation de l'intermédiaire de formule 2 : 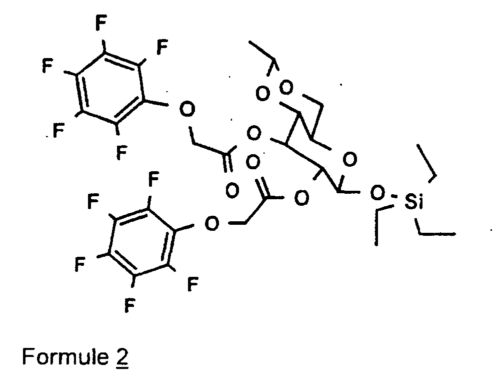 le 2,3-bis pentafluorophénoxy acétyl-4,6-éthylidène-1-O-triéthylsilyl-β-D-glucopyranoside pour le condenser avec la 4'-déméthyl épipodophyllotoxine (4'-DMEP), protégé en position 4', cette protection peut être un groupe carbobenzyloxy (Z), clivé par hydrogénolyse, un groupe aryloxyacétyle, clivé par une amine selon Protective Groups in Organic Synthesis : Th. Greene , Ed : Wiley and Sons 2^{nd} edition 1991, p 92 et 96. Parmi les groupes aryloxyacétyles protecteurs de la 4'-DMEP, on peut citer à titre d'exemples les groupes phénoxyacétyle et pentafluorophénoxyacétyle. Cette condensation s'opère en présence d'acide de Lewis. Le groupement phosphate sur la position 4' est alors introduit par le diisopropyl phosphoramidite de dibenzyle, suivi d'une oxydation, d'une hydrogénolyse et d'une salification. Les caractéristiques particulières du composé de formule 2 sont qu'il est stable, et que le groupement triéthyl silyloxy en position anomérique est en position β équatoriale uniquement.

Ce composé a la particularité d'être d'une grande stabilité, cristallin, recristallisable donc bien purifiable, et conservable dans des conditions usuelles, sans précautions particulières. La poudre obtenue peut être stockée à la température ambiante du laboratoire ou de l'atelier pendant quelques mois, voire plus, sans altération. Les rendements sont satisfaisants et le procédé est aisé à mettre en oeuvre

L'utilisation du groupement triéthylsilyle sur l'alcool anomérique est important et particulier. Il est trouvé de façon remarquable, la grande stabilité de ce groupement par rapport à son analogue triméthylsilyle. En effet, le composé de formule 2 ayant un groupement triméthylsilyle en position anomérique, ne se conserve pas. Sa sensibilité chimique le rend impropre à une utilisation industrielle pratique. Il est instable en présence de silice, dans les conditions du suivi des réactions par ccm. Le groupement triméthylsilyle s'hydrolyse pour redonner son composé de départ : le mélange des deux alcools anomères. Ce phénomène est essentiel d'un point de vue industriel quand l'opération s'effectue sur des quantités de plusieurs kilos ou plus de produit à transformer.

L'intérêt de cette nouvelle méthode de préparation, outre qu'elle utilise un composé stable, comme intermédiaire clé, est que le nombre d'étapes est limité, et que l'on utilise une stratégie de synthèse convergente, plutôt que linéaire, ce qui est très économique industriellement.

La préparation du composé de formule 2 s'effectue à partir de l'éthylidène-D-glucose par estérification des 3 fonctions alcools en position 1, 2 et 3 par le chlorure de pentafluorophénoxyacétyle dans le chlorure de méthylène et la pyridine à 0°C. Une caractéristique de la synthèse est également la purification de l'acide pentafluorophénoxy acétique, qui est issu du pentafluorophénol. Ce phénol peut être contaminé par des dérivés tétrafluorés. Ainsi donner des analogues acides tétrafluorophénoxyacétiques. Cette purification se fait par recristallisation du sel de pipérazine de l'acide acide brut en quantité stoéchiométrique (1/2 mole de pipérazine pour une mole d'acide pentafluoro phénoxyacétique à purifier) dans le mélange méthanol-eau, puis acidification et filtration de l'acide pentafluorophénoxyacétique pur.

Le triester de l'éthylidène glucose ainsi obtenu est constitué par le mélange des 2 anomères α et β en position 1. Ce mélange est hydrolysé sélectivement sur la position 1 en présence d'éthanolamine dans le THF pour conduire au mélange des 2 anomères correspondants. Ce mélange est ensuite silylé par le chlorure de triéthylsilyle dans le chlorure de méthylène à 0°C en présence de triéthylamine.

Le composé, de formule 2, obtenu brut sous forme d'huile, est cristallin après une chromatographie éclair, stable et se conserve à température ambiante. La condensation de cet intermédiaire avec le 4'-pentafluorophénoxyacétyl-4'-déméthyl épipodophyllotoxine à - 20°C dans l'acetonitrile ou le chlorure de méthylène, et en présence d'éthérate de BF₃ fournit le triester.

L'hydrolyse sélective du groupe ester en 4'- fournit le diester en 2",3" et le phénol libre en position 4'. Ce composé est alors opposé au dusopropyl phosphoramidite de dibenzyle en présence de 1*H*-tétrazole, ou bien de chlorhydrate de pyridinium, dans le THF. Le phosphite obtenu est oxydé directement par l'eau oxygénée en phosphate dibenzylé. Cet intermédiaire est alors hydrogénolysé pour conduire au dérivé phosphate acide. Ce composé peut se salifier avec la N-méthyl-D-glucamine. On obtient ainsi le composé décrit dans le brevet WO 96/12727, de façon directe et plus efficace que par la méthode décrite dans ce dernier brevet.

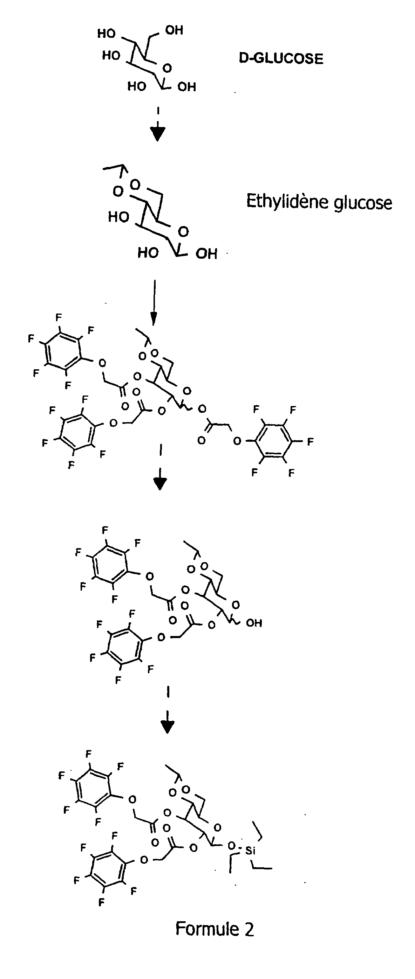

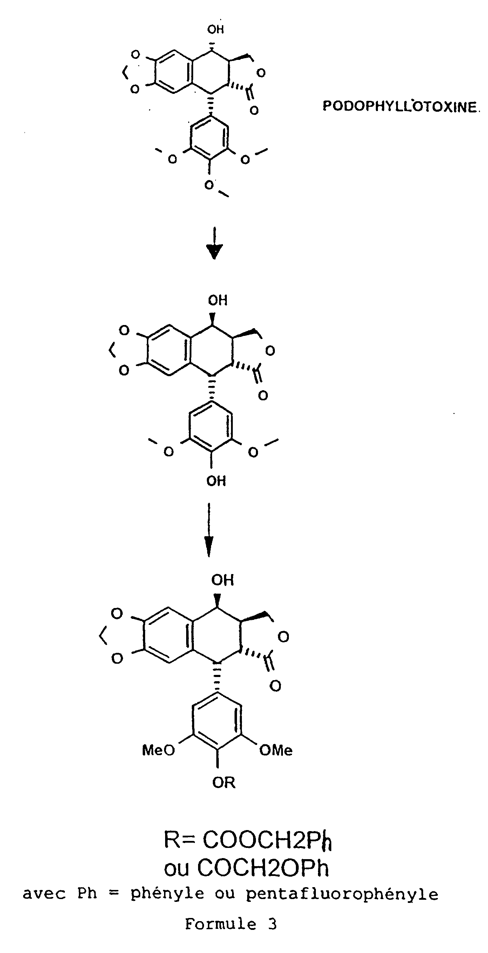

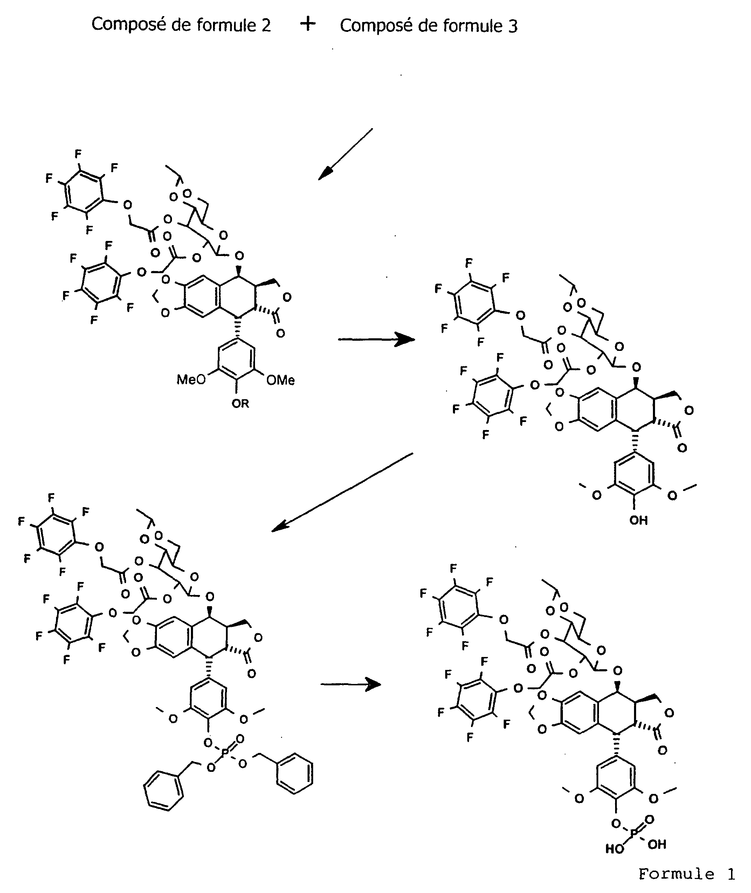

Le schéma général de synthèse du composé de formule I est illustré à la figure 1 annexée.

Les modes opératoires suivants permettent d'illustrer l'intérêt de la présente invention :

### Etape 1 : Purification de l'acide pentafluorophénoxyacétique

100 g d'acide pentafluorophénoxy acétique (contaminé par des acides tétrafluorophénoxyacétiques) sont mis en solution dans 1 L d'un mélange méthanol-eau (80/20) avec 17,8 g de pipérazine. Le sel de pipérazine cristallise sous agitation. Après filtration, ce sel est recristallisé 2 fois dans un mélange méthanol-eau (80/20) pour obtenir, après acidification, 50 g d'acide pentafluorophénoxyacétique de très grande pureté (99.7%).

### Etape 2 : 1,2,3-Tri pentafluorophénoxyacétyl-4,6-éthyldidène-D-glucopyranose.

27 mL de chlorure d'oxalyle sont ajoutés sous agitation à une solution de 70 g d'acide pentafluorophénoxyacétique en solution dans 300 mL de chlorure de méthylène et 2 mL de DMF, à température ordinaire. Après 1 h, l'évolution du gaz carbonique cesse. (Un prélèvement montre que la réaction est complète par traitement d'un aliquot dans du méthanol anhydre). Cette solution est refroidie à 0°C puis additionnée goutte à goutte à 14 g de 4,6-éthylidène-D-glucose en solution, avec 82 mL de pyridine, dans 300 mL de chlorure de méthylène. Après agitation 1 h, le milieu réactionnel est versé sur une solution d'acide chlorhydrique 1N (150 mL). La phase organique, séparée, est séchée sur sulfate de sodium, filtrée, évaporée sous vide pour fournir le triester, sous forme de mélange des deux anomères, qui est utilisé directement dans l'étape suivante.
(Système ccm : SiO₂ ; heptane-acétate d'éthyle (70/30) ; Rf = 0.4).
¹H RMN, 200 MHz, (CDCl₃): δ = 1.31 (d, 3H, H-8), 3.51 (m, 2H, H-4, H-6ax), 3.82 (m, 1H, H-5), 4.14 (dd, 1H, H-6eq), 4.65 (q, 1H, H-7), 4.66 (s, 2H, OC*H*_{*2*}CO), 4.77 (s, 2H, OC*H*_{*2*}CO), 4.84 (s, 2H, OC*H*_{*2*}CO), 5.18 (dd, 1H, H-2), 5.44 (t, 1H, H-3), 6.37 (d, 1H, H-1, J₁₋₂ = 3.7Hz).

### Etape 3 : 2,3-Dipentafluorophénoxyacétyl-4,6-éthylidène-D-glucopyranose.

8,2 mL d'éthanolamine sont ajoutés en 2 portions égales,à une heure d'intervalle, à la solution du triester de l'étape 2 précédente, dans 200 mL de THF. L'agitation est maintenue à température ambiante pendant 1 heure supplémentaire. Le milieu réactionnel est versé sur 200 mL d'HCl 1N et extrait par 2 fois 200 mL d'acétate d'éthyle. Les phases organiques sont rassemblées, séchées sur sulfate de sodium, filtrées et évaporées. Une chromatographie éclair de l'extrait brut (SiO₂, éther de pétrole-acétate d'éthyle 75/25) permet d'obtenir 31 g (70 %) du diester sous forme du mélange des alcools anomères.
(Système ccm : SiO₂ ; Toluène-acétate d'éthyle (95/5) ; Rf = 0.2).
¹H RMN, 200 MHz, (CDCl₃): δ = 1.33 (d, 3H, H-8), 3.51 (m, 3H, H-4, H-6ax, H-5), 4.20 (dd, 1H, H-6eq), 4.69 (q, 1H, H-7), 4.79 (s, 4H, OC*H*_{*2*}CO), 4.94 (m, H-2, H-1 anomer β), 5.30 (t, H-3 anomer β), 5.47 (d, H-1 anomer α), 5.59 (t, H-3 anomer α).

### Etape 4 :1-Triéthylsilyl-2,3-dipentafluorophénoxyacétyl-4,6-éthylidène-β-D-glucopyranoside

8,4 mL de chlorure de triéthylsilyle sont ajoutés goutte à goutte dans une solution de 30 g de diester précédent dans le chlorure de méthylène (250 ml) à 0°C sous agitation, en présence de 8,3 mL de triéthylamine. Après 1 h, le milieu réactionnel est jeté dans l'eau glacée et extrait par 2 fois 100 mL de chlorure de méthylène, pour conduire à 34,5 g d'une huile. Cette huile subit une chromatographie-filtration éclair sur silice, éluée par un mélange éther de pétrole-acétate d'éthyle (90/10) pour fournir 70 % du composé silylé exclusivement en anomère β. Ce composé est cristallisé (F ≤ 60°C) et stable.
(Système ccm : SiO₂; heptane-acétate d'éthyle 70/30 ; Rf = 0,6).
¹H RMN, 200 MHz: δ = 0.52 (m, 6H, Si-C*H*_{*2*}), 0.85 (m, 9H, SiCH₂C*H*_{*3*}), 1.18 (d, 3H, H-8), 3.60 (m, H-6ax, H-4, H-5), 4.02 (d, 1H, H-6eq), 4.70-5.02 (m, OC*H*_{*2*}CO, H-7, H-2), 4.89 (d, 1H, H-1, J₁₋₂ = 8.4Hz), 5.33 (t, 1H, H-3).

### 4'-Pentafluorophénoxyacétyl-4'-épipodophyllotoxine:

13,4 mL de chlorure d'oxalyle sont ajoutés sous agitation à une solution de 33,9 g d'acide pentaflùorophénoxyacétique en solution dans 300 mL de chlorure de méthylène et 1 mL de DMF, à température ordinaire. Après 1 h, l'évolution du gaz carbonique cesse. (Un prélèvement montre que la réaction est complète par traitement d'un aliquot dans du méthanol anhydre). Cette solution est refroidie à 0°C puis additionnée goutte à goutte à 40 g de 4'-DMEP en solution dans 300 mL de chlorure de méthylène à 0°C contenant 25 mL de pyridine. Après retour à la température ambiante, le milieu réactionnel est laissé sous agitation 2 h puis versé sur une solution d'acide chlorhydrique 1N. La phase organique séparée, est séchée sur sulfate de sodium anhydre, filtrée et évaporée sous pression réduite.
Le résidu brut est purifié par chromatographie éclair (chlorure de méthylène - acétone 95 :5) pour donner 44g de composé sous forme de poudre amorphe correspondant à un rendement de 70 %.C₂₉H₂₁F₅O₁₀ : 624.467.
¹H RMN: δ = 2.83 (m, 1H, H₃, J₃₋₄=3.68 Hz), 3.31 (dd, 1H, H₂,), 3.63 (s, 6H, 3'5'-OCH₃), 4.19 (dd, 1H, H₁₁ₐ), 4.35 (t,1H, H_{11b}), 4.60 (d, 1H, H₁, J₁₋₂=5.2Hz), 4.74 (dd, 1H, H₄), 5.05 (s, 2H, CH₂), 5.47 (d, OH₄ ), 6.00 (s,2H, H₁₅ₐH_{15b}), 6.33 (s, 2H, H_{2'6}'), 6.53 (s, H₈), 6.95 (s, 1H, H₅).

### Etape 5 : 4',2",3"-tripentafluorophénoxyacétyl-4",6"-éthylidène-β-D-glucopyranoside de 4'-déméthyl épipodophyllotoxine.

40 g (0,064 moles) de 4'-pentafluorophénoxyacétyl-4'-déméthyl épipodophyllotoxine sont dissous dans 300 mL de chlorure de méthylène, puis on y introduit 64 g (0.083 moles) de dérivé silylé, obtenu à l'étape 4, dissous dans 300 mL de chlorure de méthylène. La solution est refroidie à -25°C sous agitation, puis on introduit alors 29 g (0,205 moles) d'éthérate de trifluorure de bore, sous atmosphère inerte, et le milieu est maintenu pendant 3 h. Le suivi de la réaction est observé par ccm. L'hydrolyse du milieu s'effectue par de l'eau (2 fois 500 mL). La phase organique est de nouveau lavée par 400 mL d'eau, séchée sur Na₂SO₄, et concentrée pour obtenir une huile qui cristallise dans un mélange pentanol-2 / eau en donnant 60 g de produit sous forme de poudre blanche qui sera utilisé tel quel pour l'étape suivante.

### Etape 6: 2",3"-Dipentafluorophénoxyacétyl-4",6"-éthylidène-β-D-glucopyranoside de 4'-déméthyl épipodophyllotoxine.

25 g du composé de l'étape précédente mis en solution dans un mélange 300 mL de THF et 150 mL de méthanol sont traités par 25 g d'acétate d'ammonium pendant 2 h, sous agitation, à température ambiante. Après ajout de 200 mL d'acide chlorhydrique 1N (pH 6), le produit est extrait au chlorure de méthylène. La phase organique est lavée à l'eau, séchée sur sulfate de sodium anhydre, filtrée puis évaporée sous pression réduite pour donner une huile qui cristallise dans l'éther isopropylique sous forme de poudre blanche (18 g, soit 83 % de rendement). Le produit est recristallisé dans l'éthanol avec un rendement de 75 % pour donner 13.4 g de produit pur.
C45H34F10O17 (1036.75). F : 164°C.
¹H RMN, 400 MHz: δ = 1.21(d, 3H, H-8"), 2.92 (m, 1H, H-3), 2.97(dd, 1H, H-2), 3.61 (s, 6H, 3',5'-OCH₃), 3.66 (m, 3H, H-4", H-5", H-6"ax), 4.15 (d, 1H, H-6"eq), 4.18 (t, 1H, H-11b), 4.22 (d, 1H, H-10"b), 4.30 (t, 1H, H-11a), 4.41 (d, 1H, H-1), 4.73 (d, 1H, H-10"b), 4.76 (m, 1H, H-7"), 4.85 (t, 1H, H-2"), 4.92 (d, 1H, H-4), 4.98 (d, 2H, H-10"a), 5.28 (d, 1H, H-1", J_{1"-2"} = 8 Hz), 5.39 (t, 1H, H-3"), 5.93 (s, 1H, H-15b), 6.04 (s, 1H, H-15a), 6.15 (s, 2H, H-2',6'), 6.47 (s, 1H, H-8), 7.06 (s, 1H, H-5), 8.28 (s, 1H, 4'-OH).

### Etape 7: 2",3"-Dipentafluorophénoxyacétyl-4",6"-éthylidène-β-D-glucopyranoside de 4'-déméthyl-4'-dibenzylphosphate épipodophyllotoxine.

1,35 g de 1*H*-tétrazole anhydre sont ajoutés à une solution de 5 g de composé de l'étape précédente et 3,3 g de diisopropyl phosphoramidite de dibenzyle dans 75 mL de THF sous azote, à température ordinaire. Après 15 mn de réaction, le milieu est refroidi à 0°C et 5 mL d'une solution d'eau oxygénée à 30 % sont introduits. Cette solution est laissée sous agitation pendant 10 mn à cette température, puis jetée sur une solution de thiosulfate de sodium et extraite par de l'éther éthylique (2 fois 100 mL). Après séchage sur sulfate de sodium et filtration, puis évaporation sous vide, on obtient 7 g d'une huile. Cette huile est purifiée par chromatographie éclair sur silice (élution : heptane-acétate d'éthyle 50/50) pour fournir 6,1 g de produit pur (R^{dt} 97 %). Ce composé est cristallisé F = 88°C.
¹H RMN 400MHz: δ = 1.21(d, 3H, H-8"), 2.91 (m, 1H, H-3), 3.03 (dd, 1H, H-2), 3.62 (s, 6H, 3',5'-OCH₃), 3.68 (m, 3H, H-4", H-5", H-6"ax), 4.10-4.32 (m, 4H, H-6"eq, H-11a, H-11b, H-10"b), 4.53 (d, 1H, H-1), 4.73 (d, 1H, H-10"b), 4.76 (m, 1H, H-7"), 4.85 (t, 1H, H-2"), 4.93 (d, 1H, H-4), 4.97(d, 2H, H-10"a),5.17 (d, 4H, C*H2*-C6H5), 5.28 (d, 1H, H-1", J_{1"-2"} = 7.86 Hz), 5.38 (t, 1H, H-3"), 5.94 (s, 1H, H-15b), 6.04 (s, 1H, H-15a), 6.26 (s, 2H, H-2,'6'), 6.51 (s, 1H, H-8), 7.07 (s, 1H, H-5), 7.38 (m, 10H, arom.).

Le tétrazole peut être remplacé par le chlorhydrate de pyridinium anhydre pour conduire de façon identique au même composé dibenzylphosphate : A une solution de 136 g du composé obtenu à l'étape 6 précédente, dans 1,5 L de THF, sont ajoutés successivement 87 mL de diisopropyl phosphoramidite de dibenzyle et 45 g de chlorhydrate de pyridinium anhydre. Après 1 h d'agitation, à température ordinaire, le milieu réactionnel est refroidi et 136 mL d'une solution d'eau oxygénée à 32 % est ajoutée. Le milieu réactionnel est encore agité 1 h à cette température. On ajoute ensuite une solution de thiosulfate de sodium et le milieu réactionnel est extrait par de l'éther éthylique. Un traitement chromatographique identique fournit le dérivé dibenzylphosphate recherché avec un rendement de 65 %.

### Etape 8: 2",3"-dipentafluorophénoxyacétyl-4",6"-éthylidène-β-D-glucopyranoside de 4'-phosphate-4'-déméthyl épipodophyllotoxine.

136 g du composé dibenzylphosphate obtenu selon l'étape précédente sont placés en solution dans 250 mL d'éthanol et 500 mL d'acétate d'éthyle en présence de 10 g de charbon palladié à 10 %. L'ensemble est agité sous atmosphère d'hydrogène pendant 30 mn. Le catalyseur est ensuite filtré et le filtrat évaporé sous pression réduite pour fournir une poudre amorphe en tant que phosphate libre, avec un rendement de 98 %.

Formation du sel de N-méthylglucamine : Addition d'une solution de 17 g de N-méthyl-D-glucamine en solution dans 39 mL d'eau à une solution de 50 g de dérivé phosphate libre précédent, dans un mélange de 311 mL d'acétone et 830 mL d'éthanol, sous agitation. On obtient, après filtration, des cristaux blancs qui sont lavés par de l'heptane. Après séchage, on obtient 61 g (R^{dt} 91 %) du sel de N-méthyl-D-glucamine, ayant un point de fusion de 130°C.
(Système ccm ; SiO₂ ; chlorure de méthylène-méthanol-acide acétique = 90110/5 ; Rf = 0.5).
Analyse élémentaire : C₄₅H₃₅F₁₀O₂₀P,2(C₇H₁₇NO₅),1H₂O
PM = 1525,17 Calc : C% 47,02 ; H% 4,61 ; N% 1,86
Trouvé (1,1%H₂O) : C% 47,18 ; H% 4,62 ; N% 1,84.
¹H RMN, 400 MHz: δ = 1.18 (d, 3H, H-8"), 2.29 (s, 6H, N-CH3),2.50-2.80 (m, 6H, glucamine), 2.90 (m, 1H, H-3), 2.95 (dd, 1H, H-2), 3.00-3.50 (m, 20H, glucamine), 3.57 (s, 6H, 3',5'-OCH₃), 3.63 (m, 3H, H-4", H-5", H-6"ax), 3.79 (s, 2H, glucamine), 4.15 (d, 1H, H-6"eq), 4.19 (t, 1H, H-11b), 4.24 (d, 1H, H-10"b), 4.32 (t, 1H, H-11a), 4.43 (d, 1H, H-1), 4.72 (d, 1H, H-10"b), 4.74 (m, 1H, H-7"), 4.84 (t, 1H, H-2"), 4.93 (d, 1H, H-4), 4.96 (d, 2H, H-10"a), 5.25 (d, 1H, H-1", J_{1"-2"} = 7.84 Hz), 5.37 (t, 1H, H-3"), 5.92 (s, 1H, H-15b), 6.03 (s, 1H, H-15a), 6.12 (s, 2H, H-2',6'), 6.47 (s, 1H, H-8), 7.05 (s, 1H, H-5).

## Revendications

1. Procédé de préparation, du 4'-déméthyl-4'-phosphate-2".,3"-bispentafluorophénoxyacétyl-4",6"-éthylidène-β-D-glucoside d'épipodophyllotoxine de formule 1 ainsi que de ses sels, en particulier du sel de N-méthyl-D-glucamine 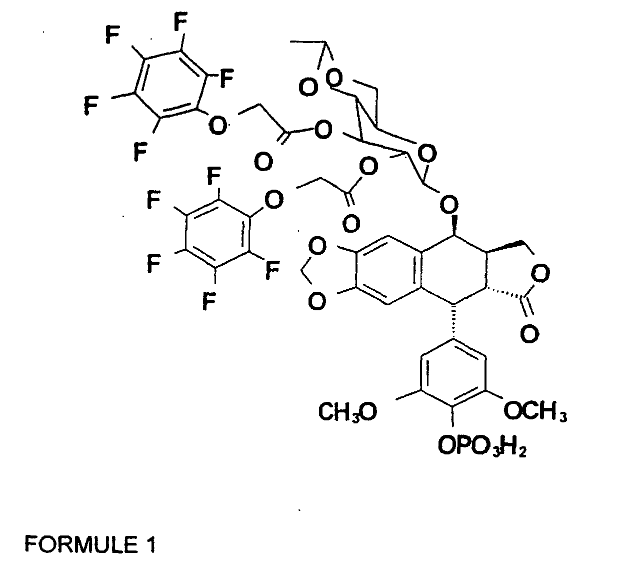 **caractérisé en ce qu'**il implique la condensation du 2,3-bis pentafluorophénoxy acétyl-4,6-étriylidène-1-O-triéthylsilyl-β-D-glucopyranoside, de formule 2 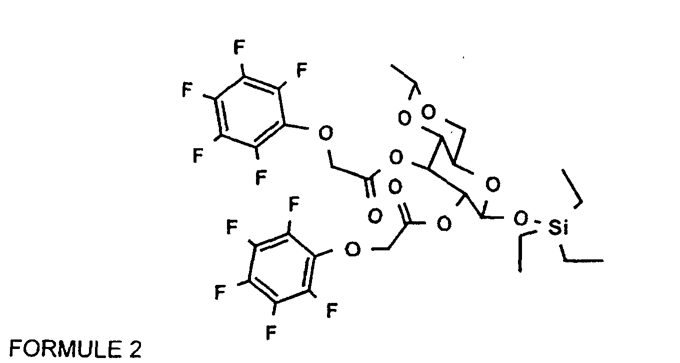 avec la 4'-déméthyl-4'-aryloxyacétyl-épipodophyllotoxine de formule 3 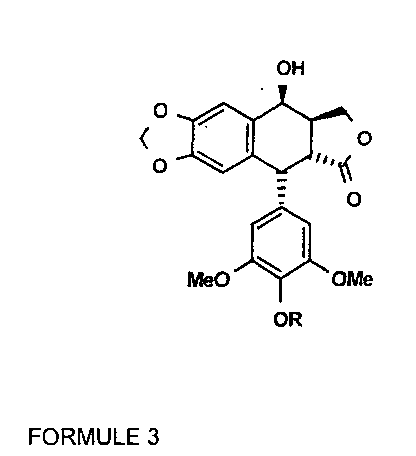 Dans laquelle R représente un groupe aryloxyacétyle, tel que phénoxyacétyle et pentafluorophényloxyacétyle.

2. Composé de formule 2 ci-dessous, **caractérisé en ce que** la position anomère est sous forme exclusivement β.

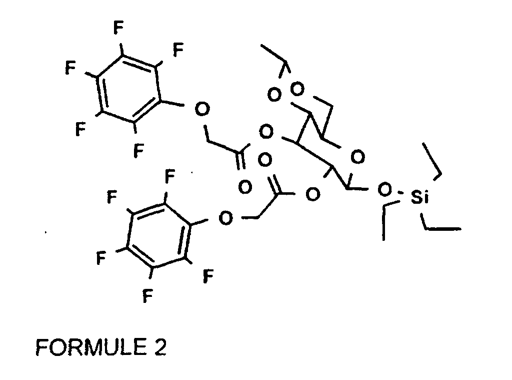

3. Procédé de préparation d'un composé de formule 2 selon la revendication 2, **caractérisé en ce que** l'on forme exclusivement l'anomère β à partir du 2,3-dipentafluorophénoxyocétyl-4,6-éthylidène-D-glucopyranose sous forme du mélange des deux alcools anomères, par réaction avec le chlorure de triéthylsilyle.

## Patentansprüche

1. Verfahren zur Herstellung des 4'-Demethyl-4'-phosphat-2'',3''-bispentafluorphenoxyacetyl-4'',6''-ethyliden-β-Dglucosids von Epipodophyllotoxin der Formel 1 sowie von dessen Salzen, insbesondere des N-Methyl-D-glucaminsalzes 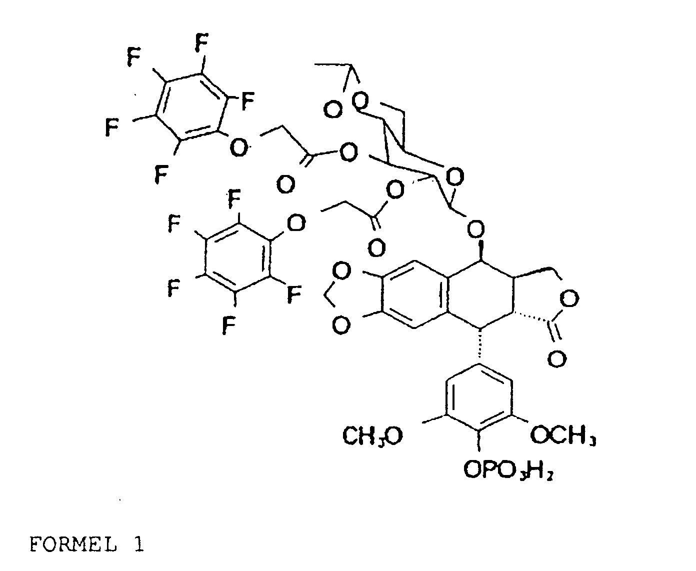 **dadurch gekennzeichnet, dass** es die Kondensation des 2,3-Bispentafluorphenoxyacetyl-4,6-ethyliden-1-O-triethylsilyl-β-Dglucopyranosids der Formel 2 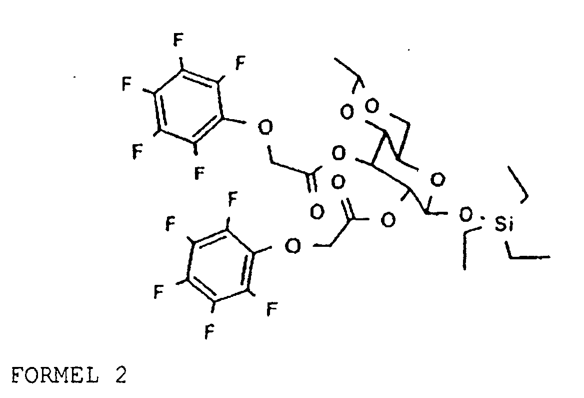 mit 4'-Demethyl-4'-aryloxyacetylepipodophyllotoxin der Formel 3 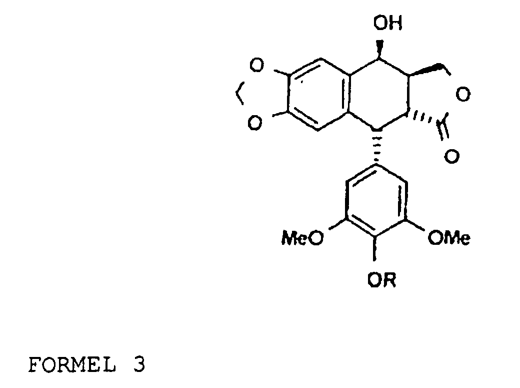 in welcher R für eine Aryloxyacetylgruppe, wie Phenoxyacetyl und Pentafluorphenyloxyacetyl, steht, umfasst.

2. Verbindung der nachstehenden Formel 2, **dadurch gekennzeichnet, dass** die Anomerieposition ausschließlich in β-Form vorliegt

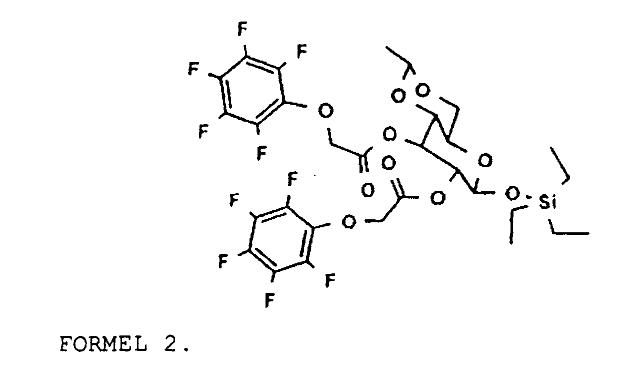

3. Verfahren zur Herstellung einer Verbindung der Formel 2 nach Anspruch 2, **dadurch gekennzeichnet, dass** man ausgehend von 2,3-Dipentafluorphenoxyacetyl-4,6-ethyliden-D-glucopyranose in Form der Mischung der zwei anomeren Alkohole durch Umsetzung mit Triethylsilylchlorid ausschließlich das β-Anomer bildet.

## Claims

1. Process for preparing epipodophyllotoxin 4'-demethyl-4'-phosphate-2",3"-bis(pentafluorophenoxyacetyl)-4",6"-ethylidene-β-D-glucoside of formula 1 and also salts thereof, in particular the N-methyl-D-glucamine salt 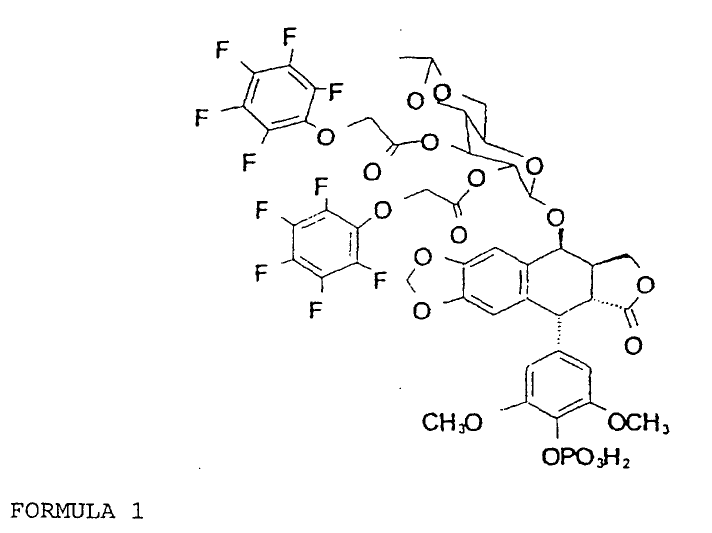 **characterized in that** it involves the condensation of 2,3-bis(pentafluorophenoxy)acetyl-4,6-ethylidene-1-O-triethylsilyl-β-D-glucopyranoside, of formula 2 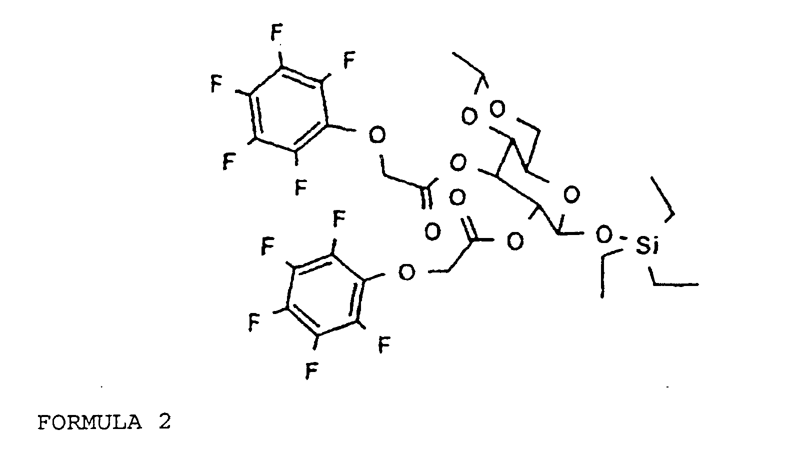 with 4'-demethyl-4'-aryloxyacetyl epipodophyllotoxin of formula 3 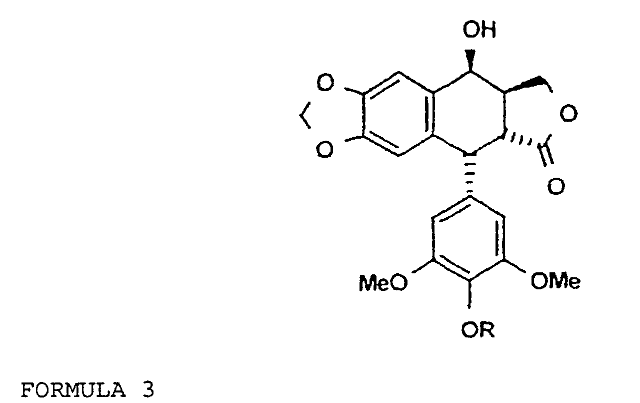 in which R represents an aryloxyacetyl group, such as phenoxyacetyl or pentafluorophenoxyacetyl.

2. Compound of formula 2 below, **characterized in that** the anomeric position is in exclusively β-form.

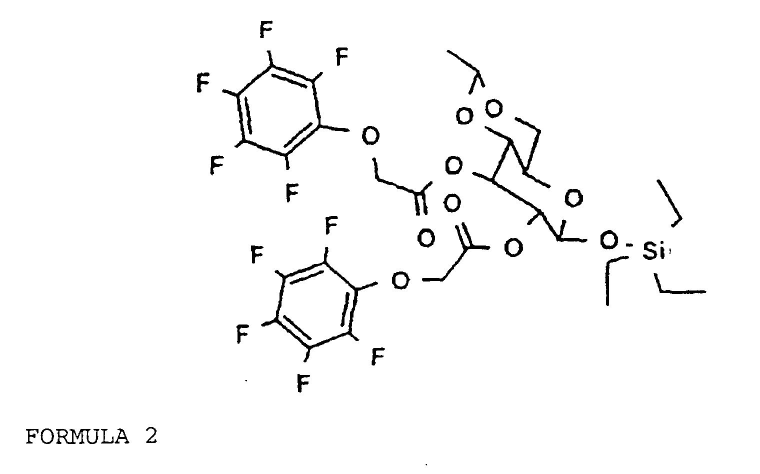

3. Process for preparing a compound of formula 2 according to Claim 2, **characterized in that** the β anomer is formed exclusively from 2,3-dipentafluorophenoxyacetyl-4,6-ethylidene-D-glucopyranose in the form of a mixture of the two anomeric alcohols, by reaction with triethylsilyl chloride.
